# FASCICULE DE BREVET EUROPEEN

(11) **EP 2 150 127 B1**
(45) Date de publication et mention de la délivrance du brevet: **06.06.2018**
(21) Numéro de dépôt: 08805690.8
(22) Date de dépôt: 23.04.2008
(51) Int. Cl.: A23L 21/20, C11B 1/10

(54) **PROCEDE DE TRAITEMENT DE LA PROPOLIS**
VERFAHREN ZUR BEHANDLUNG VON PROPOLIS
PROPOLIS TREATMENT METHOD

(30) Priorité: 24.04.2007 FR 0754648
(43) Date de publication de la demande: 10.02.2010
(73) Titulaire: ABEILLES SANTE, 65110 Cauterets (FR)
(72) Inventeur: BALLOT-FLURIN, Catherine, F-65700 Lahitte Toupiere (FR)
(74) Mandataire: Fragnaud, Aude
(86) Numéro de dépôt international: PCT/FR2008/050736
(87) Numéro de publication internationale: WO 2008/145926

(56) Documents cités:
- EP-A- 0 109 993
- WO-A-02/062362
- WO-A-2005/094853
- DE-A1- 4 320 315
- DE-U1-202006 012 266
- FR-A- 2 374 030
- FR-A- 2 594 336
- FR-A- 2 837 105
- DATABASE WPI Week 198239 Thomson Scientific, London, GB; AN 1982-83128E XP002457214 & SU 884 703 B (DOMEST CHEM GOODS) 30 novembre 1981 (1981-11-30)
- DATABASE WPI Week 198701 Thomson Scientific, London, GB; AN 1987-002051 XP002457215 & HU 40 010 A (BENCZE P) 28 novembre 1986 (1986-11-28)
- DATABASE WPI Week 200351 Thomson Scientific, London, GB; AN 2003-536144 XP002457216 & JP 2003 061593 A (API KK) 4 mars 2003 (2003-03-04)
- DATABASE WPI Week 200604 Thomson Scientific, London, GB; AN 2006-032290 XP002457217 & JP 2005 336137 A (NIPPON PROPOLIS KK) 8 décembre 2005 (2005-12-08)
- DATABASE WPI Week 199339 Thomson Scientific, London, GB; AN 1993-309087 XP002457218 & JP 05 221819 A (SAWADA Y) 31 août 1993 (1993-08-31)
- DATABASE CAPLUS [Online] CHEMICAL ABSTRACTS SERVICE, COLUMBUS, OHIO, US; XP002457348 extrait de STN Database accession no. 1911:4349
- DATABASE EPODOC EUROPEAN PATENT OFFICE, THE HAGUE, NL; XP002457213 & JP 08 143462 A (SAN CREST KK; SUZUKI IKUKATSU) 4 juin 1996 (1996-06-04)
- DATABASE WPI Week 199924 Thomson Scientific, London, GB; AN 1999-280734 XP002457219 & JP 10 338641 A (KASSEI SHOKUHIN KENKYU CENT KK) 22 décembre 1998 (1998-12-22)
- DATABASE FSTA [Online] INTERNATIONAL FOOD INFORMATION SERVICE (IFIS), FRANkFURT-MAIN, DE; 2003, SEUNG-KWAN HAN: "Antioxidative effect of different kinds of propolis on the oxidation of edible oils." XP002508303 Database accession no. 2003-00-l0565 & KOREAN JOURNAL FOR FOOD SCIENCE OF ANIMAL RESOURCES, vol. 23, no. 2, 2003, pages 168-171,
- DATABASE WPI Week 200538 Thomson Scientific, London, GB; AN 2005-370109 XP002508304 & KR 2005 004 419 A (HAN S K) 12 janvier 2005 (2005-01-12)
- DATABASE WPI Week 200262 Thomson Scientific, London, GB; AN 2002-581545 XP002508305 & RU 2 185 181 C1 (TENTORIUM CO LTD) 20 juillet 2002 (2002-07-20)
- DATABASE WPI Week 198402 Thomson Scientific, London, GB; AN 1984-009467 XP002508306 & RO 70 344 A (INST NICOLAU VIRUSO) 5 juin 1980 (1980-06-05)
- DATABASE WPI Week 198307 Thomson Scientific, London, GB; AN 1983-16690K XP002508307 & RO 79 518 A (INTR MEDICAMENTE BIOFARM) 30 juillet 1982 (1982-07-30)

## Description

L'invention concerne un procédé de traitement de la propolis. La fabrication de produits d'hygiène, de soin, de cosmétiques et de produits alimentaires et compléments alimentaires comprenant un ou des extraits de propolis obtenu (s) par le procédé est décrite ci-dessous. On rappelle que la propolis désigne toute une série de substances résineuses, gommeuses et balsamiques, de consistance visqueuse. Ces substances sont recueillies sur certaines parties (bourgeons et écorces essentiellement) de végétaux (certains arbres principalement) par les abeilles, qui les rapportent à la ruche et qui les additionnent et les modifient en partie par l'apport de certaines de leurs sécrétions propres (cire et sécrétions salivaires essentiellement).

La propolis est utilisée à de nombreuses fins à l'intérieur de la ruche :
- pour construire éventuellement de véritables barrières de défense ;
- pour rendre la ruche parfaitement hermétique, permettant une bonne isolation thermique ;
- pour vernisser l'ensemble des surfaces intérieures afin d'en supprimer les aspérités ;
- pour recouvrir d'une fine pellicule les nouveaux rayons ainsi que l'intérieur de la totalité des cellules avant que la reine ne vienne y pondre, ce qui constitue une désinfection efficace (sorte de « stérilisation »);
- enfin, pour enduire, combinée avec de la cire, les petits animaux ou insectes, qui ne peuvent être évacués, sorte d'embaumement s'opposant ainsi à toute décomposition putride.

Il est reconnu par la communauté scientifique que la propolis est une substance possédant des propriétés :
- antibiotiques importantes et étendues à de nombreuses souches microbiennes, de plus l'extrait de propolis augmente l'efficacité de certains antibiotiques (potentialise). D'autre part, les expériences ont montré que les souches de microbes pathogènes sont beaucoup plus sensibles à l'action de la propolis qu'avec des antibiotiques classiques: penicilline, tetracycline, ampicilline, monomycine,
- antivirales,
- antifongiques,
- antigerminatives,
- anesthésiques très puissantes et supérieures entre autres à la cocaïne dont elle n'a pas de surcroît les inconvénients sur le plan des effets secondaires;
- cicatrisantes notables par stimulation de la régénération tissulaire;
- action sur le métabolisme du neurone,
- antioxydantes,
auxquelles il faut ajouter encore des propriétés anti-inflammatoires non négligeables et une influence heureuse sur certains mécanismes immunologiques qui entraîne un renforcement du terrain contre l'agression en général.

Il existe à ce jour de nombreuses présentations dont beaucoup sous forme de spécialités où la propolis est :
- soit seule substance active, sous sa forme purifiée (c'est-à-dire débarrassée de toutes les impuretés mécaniques diverses dont elle a pu être souillée dans la ruche : fibres de bois, poils d'abeilles, etc.);
- soit en association (toujours sous sa forme purifiée) avec d'autres produits qui sont généralement médicamenteux ou diététiques.

C'est ainsi qu'on trouve en résumé et le plus souvent à l'heure actuelle :
- la propolis à l'état naturel, purifiée et seule substance active sous forme :
   - solide : pâte à mâcher ou fragments (de grosseurs variables), granulés et poudre (cette dernière pouvant être en gélules ou comprimés à avaler),
   - d'extrait dilué dans l'alcool: solution hydro alcoolique de propolis à des taux variables (de 3 à 30% selon indications, avec une moyenne de 15 à 20%),
- la propolis purifiée en association avec:
   - des substances médicamenteuses diverses qui viennent compléter l'action de la propolis dans certaines indications particulières,
   - de pommade ou d'onguent, où la propolis est en général associée à la vaseline ou à la lanoline (ou les deux à la fois) et cela dans des pourcentages variables oscillant en général de 10 à 30% (avec une moyenne de 15%),
   - des produits diététiques qui sont le plus souvent : miel, pollen ou gelée royale ou des produits non apicoles (plantes),
   - des cosmétiques.

Les documents database WPI week 198239 Thomson scientific, London, GB, AN1982-83128 XP002457214, 30/11/1981 ; database WPI week 199924 Thomson scientific, London, GB, AN 1999_280734 XP002457219, 22/12/1998 ; WO02/062362 et EP0109993 décrivent un procédé d'extraction de la propolis en une seule étape avec une solution hydro-alcoolique, c'est à dire contenant un mélange de solvants eau et alcool.

Le document DE 20 2006 012266 décrit un procédé d'extraction de la propolis avec un solvant alcoolique.

Le document database WPI week 198701 Thomson scientific, London, GB, AN1987-002051 XP002457215, 28/11/1986 décrit un procédé de traitement de la propolis consistant en deux étapes successives d'extraction, les deux extractions étant menées avec une solution hydro-alcoolique dans des proportions eau/alcool différentes.

Le document database WPI week 200351 Thomson scientific, London, GB, AN2003-536144 XP002457216, 04/03/2003 décrit un procédé de traitement de la propolis consistant en deux étapes successives d'extraction, la première extraction étant menée avec de l'éthanol et la deuxième avec de l'eau. Un additif est ensuite ajouté à l'extrait aqueux obtenu.

De même, le document FR2 594 336 décrit un procédé de traitement de la propolis consistant en deux étapes successives, la première avec de l'alcool, la deuxième avec de l'eau.

Le document database WPI week 200604 Thomson scientific, London, GB, AN2006-032290 XP002457217, 08/12/2005 décrit un procédé d'extraction de propolis avec une solution mixte d'eau, de composé organique et d'un émulsifiant.

Le document database WPI week 199339 Thomson scientific, London, GB, AN1993-309087 XP002457218, 31/08/1993 décrit une extraction de propolis avec de l'alcool et une extraction de propolis avec de l'eau ionisée. Ces deux extractions sont réalisées de manière indépendante l'une de l'autre et les résultats de ces deux extractions indépendantes sont mélangés après évaporation du surnageant des solutions.

Le document database WPI EPODOC, La Hague XP002457213 décrit un procédé de traitement de la propolis consistant à réaliser deux extractions successives avec le même solvant, à savoir de l'eau.

Le document FR2 837 105 décrit un procédé d'extraction de la propolis avec de l'huile essentielle de lavande.

Le document FR2 374 030 décrit un procédé d'extraction de la propolis avec de l'eau et en utilisant des sels métalliques comme additifs qui permettent d'aider la dissolution de la propolis dans l'eau.

Enfin, les documents référencés database FSTA (IFIS) DE 2003 SEUNG-KWAN HAN « antioxidative effect of different kinds of propolis on the oxidation of edible oils » XP002508303 N°2003-00-I0565 ; database WPI Week 200538 Thomson scientific, London, GB, AN2005-370109 XP002508304, 12/01/2005 ; database WPI Week 200262 Thomson scientific, London, GB, AN2002-581545 XP002508305, 20/07/2002 ; database WPI Week 198402 Thomson scientific, London, GB, AN1984-009467 XP002508306, 05/06/1980; database WPI Week 198307 Thomson scientific, London, GB, AN1983-16690K XP002508307, 30/07/1982 ; décrivent chacun un procédé d'extraction de la propolis avec de l'eau bouillante.

La présente invention a pour objet un procédé de traitement naturel de la propolis permettant d'obtenir une nouvelle présentation de la propolis sous forme d'extrait conservant toutes les composantes actives de la propolis brute, aucun solvant mélangé à un autre solvant ou à tout autre produit n'étant utilisé pour effectuer une extraction.

La présente invention a plus particulièrement pour objet un procédé de traitement naturel de la propolis à l'aide uniquement de solvants biologiques purs compatibles avec la certification biologique. Ce procédé permet de disposer de plusieurs types d'extraits facilement solubles et miscibles dans tout type de formules (huileuses, aqueuses, alcooliques). Le procédé permet ainsi de multiples possibilités d'applications (crèmes, baumes, préparations diététiques), tous les types d'extraits utilisés comme ingrédients dans des produits comestibles et applicables par voie cutanée et ingérables par voie orale ou par voie nasale. L'invention concerne plus particulièrement un procédé de traitement de la propolis caractérisé en ce qu'il comprend plusieurs étapes d'extractions successives opérées à partir d'un même lot de propolis, l'ensemble desdites étapes d'extraction étant réalisé sur une durée totale d'au moins 52 jours, lesdites étapes d'extraction étant réalisées strictement dans l'ordre suivant::
- A] une première étape d'extraction aqueuse, d'une durée de l'ordre de 30 jours à une température inférieure à 0°C, avec de l'eau pure, c'est-à-dire sans mélange avec un autre solvant, effectuée sur un lot de propolis brute afin d'obtenir un premier extrait aqueux ayant des premières propriétés antibactériennes, antiseptiques et isotoniques c'est-à-dire compatibles avec une application sur les muqueuses et applicable directement sur la peau,
- B] une deuxième étape d'extraction avec de l'alcool pur, ladite deuxième extraction alcoolique étant réalisée sur des résidus de filtration obtenus à l'issue de ladite première étape d'extraction aqueuse, par macération et brassage manuel discontinu desdits résidus de filtration aqueux pendant une durée d'au moins 15 jours, de manière à obtenir un deuxième extrait alcoolique ayant des deuxièmes propriétés,
- C] une troisième étape d'extraction avec de l'huile pure, ladite troisième extraction huileuse étant réalisée sur des résidus de filtration obtenus à l'issue de ladite deuxième étape d'extraction alcoolique, en les faisant macérer dans l'huile pendant au moins une semaine, de manière à obtenir un troisième extrait huileux ayant des troisièmes propriétés.

La deuxième extraction effectuée avec un deuxième solvant pur de nature différente du premier est réalisée sur les résidus de filtration obtenus à l'issue de l'extraction réalisée avec le premier solvant, de manière à récupérer et rendre utilisable la totalité des composantes de la propolis en utilisant en outre que des solvants biologiques. Enfin, la troisième extraction successive à la deuxième, est effectuée sur les résidus de filtration obtenus après cette deuxième extraction, avec un troisième solvant pur différent des deux autres. Le premier solvant est de l'eau, le deuxième est de l'alcool et le troisième est de l'huile. La fabrication d'extraits de propolis pour une utilisation dans la fabrication de produits en particulier d'hygiène, de soins et de cosmétiques, alimentaires et compléments alimentaires est décrite ci-dessous. Ainsi, selon une première caractéristique, le procédé de traitement de la propolis comprend une étape d'extraction avec de l'eau pure, c'est-à-dire sans mélange avec un autre solvant, effectuée sur un lot de propolis brute afin d'obtenir un premier extrait ayant des premières propriétés. L'extrait obtenu est un extrait aqueux dont les propriétés sont des propriétés antibactériennes et isotoniques c'est-à-dire compatibles avec une application sur les muqueuses et applicable directement sur la peau.

Selon une autre caractéristique l'extraction de propolis avec de l'eau est réalisée par décoction de la propolis en mettant la propolis dans de l'eau en la portant à ébullition et/ou par infusion en laissant reposer la propolis dans l'eau portée à ébullition. Contrairement à tous les préjugés on obtient ainsi un extrait qui possède une forte activité, les matières actives de la propolis étant conservées.

Selon une autre caractéristique de l'invention, le procédé comprend une deuxième étape d'extraction avec de l'alcool pur l'extraction avec l'eau étant réalisée sur le lot de propolis brute et l'extraction avec l'alcool étant réalisée sur les résidus de filtration obtenus à l'issue de l'extraction aqueuse, de manière à obtenir le premier extrait de propolis ayant les premières propriétés puis un deuxième extrait ayant des deuxièmes propriétés, ce dernier étant débarrassé de la majorité des impuretés. Le premier extrait aqueux de propolis a des propriétés isotoniques c'est-à-dire compatibles avec une application sur les muqueuses (nasales, buccales, vaginales) et utilisable en application directe sur la peau ou pouvant être consommé. Le deuxième extrait est un extrait alcoolique de plus grande pureté (moins d'impuretés) que les extraits alcooliques existants.

L'extraction alcoolique comprend alors les étapes suivantes :
1- On fait macérer et on brasse manuellement en discontinu pendant au moins 15 jours, les résidus de filtration issus de l'étape d'extraction aqueuse, dans de l'alcool,
2- On récupère après une ou plusieurs filtration(s) les résidus de filtration,
3- On récupère l'extrait alcoolique obtenu.
Pour obtenir un extrait total c'est-à-dire un extrait ne contenant plus d'impureté et 100% des composants actifs de la propolis, on procède à trois extractions successives, le troisième solvant est de l'huile, le troisième extrait étant un extrait huileux, la première extraction étant faite à partir d'un premier lot de propolis et les extractions successives étant faites à chaque fois à partir des résidus de filtration obtenus à l'issue de l'extraction précédente. Le procédé permet ainsi de récupérer et de rendre utilisable la totalité des composantes de la propolis. Afin d'assurer une bonne pénétration de l'eau dans la propolis brute, la première étape du procédé consiste à maintenir la propolis à une température inférieure à zéro degré pendant une durée relativement longue par exemple 30 jours.

L'étape suivante consiste à effectuer une décoction de la propolis. La propolis est placée dans une eau portée à ébullition et dont la température est maintenue pendant une durée suffisamment longue mais de préférence ne dépassant pas 20 minutes, on laisse ensuite refroidir et reposer le mélange.

Le repos du mélange permet d'obtenir la séparation de la cire contenue dans la propolis, et de récupérer l'extrait à l'issue d'une ou plusieurs filtrations.

Les résidus de filtration sont conservés pour la ou les extractions suivantes.

La deuxième extraction comprend une macération et un brassage manuel en discontinu et quotidiennement, des résidus de filtration issus de la première extraction, dans de l'alcool, pendant une durée d'au moins quinze jours, puis une ou plusieurs filtration(s) de l'extrait pour obtenir l'extrait alcoolique purifié et des résidus de filtration. Lors des trois extractions successives, permettant d'obtenir un extrait total,
- la première extraction consiste à :
   - Réaliser une extraction aqueuse longue de propolis pour obtenir un extrait aqueux de propolis et des résidus de filtration, en maintenant la propolis à une température inférieure à zéro degré pendant une durée relativement longue, 30 jours par exemple puis, en effectuant une décoction de la propolis, la propolis étant placée dans de l'eau portée à ébullition pendant une durée ne dépassant pas 20 minutes, et enfin en laissant reposer au moins 24h,
- la deuxième extraction consiste à :
   - Réaliser une extraction alcoolique longue à partir des résidus de filtration issus de l'extraction aqueuse pour obtenir un extrait alcoolique de propolis et des résidus de filtration, en faisant macérer et en brassant manuellement en discontinu pendant au moins 15 jours, les résidus de filtration dans de l'alcool,
- la troisième extraction consiste à :
   - Réaliser une extraction huileuse longue à partir des résidus de l'extraction alcoolique pour obtenir un extrait huileux de propolis et des résidus de filtration, en faisant macérer dans l'huile pendant au moins une semaine les résidus de l'extraction alcoolique.
Les extraits peuvent être utilisés dans la fabrication de produits d'hygiène, de santé, de cosmétiques, alimentaires et compléments alimentaires. Un produit cosmétique comprenant, dans sa composition, l'un au moins des extraits aqueux et/ou alcoolique et/ou huileux obtenus à l'issue de l'une au moins des étapes d'extraction du procédé de traitement selon l'invention est ainsi décrit, comme par exemple: Un déodorant corporel comprenant dans sa composition au moins 50% d'extrait aqueux de propolis obtenu selon le procédé de la présente invention; Un produit d'hygiène nasale comprenant dans sa composition 50% d'extrait aqueux de propolis obtenu selon le procédé de la présente invention; Un produit d'hygiène auriculaire constitué de 100% d'extrait de propolis aqueux obtenu selon le procédé de la présente invention; Un produit d'hygiène intime comprenant dans sa composition 5% d'extrait alcoolique de propolis obtenu après une deuxième extraction selon le procédé; Une crème de soin après rasage, comprenant un complexe d'extrait de propolis comportant dans sa composition 14% d'extrait huileux et de 10% d'extrait aqueux obtenus par le procédé; Un produit anti-oxydant pour le contour des yeux ayant un effet lisse cerne et ridules, comprenant un complexe d'extrait de propolis comportant dans sa composition 14% d'extrait huileux et 10% d'extrait aqueux obtenus par le procédé selon l'invention; Un produit anti-oxydant pour le soin du corps ayant un effet hydratant et protecteur, comprenant un complexe d'extrait de propolis comprenant dans sa composition 8% d'extrait huileux de propolis et 12% d'extrait aqueux de propolis obtenus par le procédé selon l'invention; Une crème douche pour corps, visage et cheveux, comprenant dans sa composition 1 % d'extrait alcoolique obtenu par le procédé selon l'invention; Un shampoing de soin régénérant, comprenant 2,5% dans sa composition d'extrait alcoolique obtenu par le procédé selon l'invention; Un dentifrice, comprenant dans sa composition, au moins 3% d'extrait alcoolique de propolis obtenu par le procédé selon l'invention.

D'autres particularités et avantages de l'invention apparaîtront clairement à la lecture de la description qui est faite ci-après et qui est donnée à titre d'exemple illustratif et non limitatif et en regard des figures sur lesquelles :

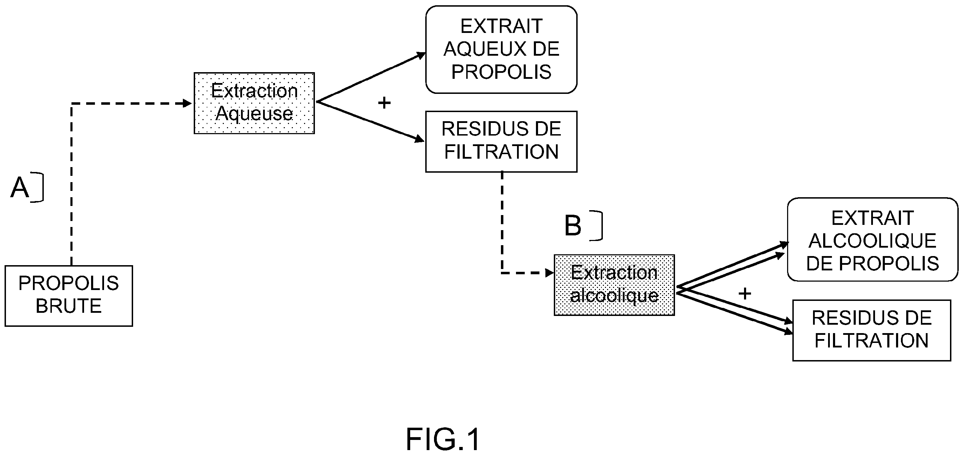

- la figure 1 représente les étapes mises en oeuvre selon un procédé ne faisant pas partie de l'invention,

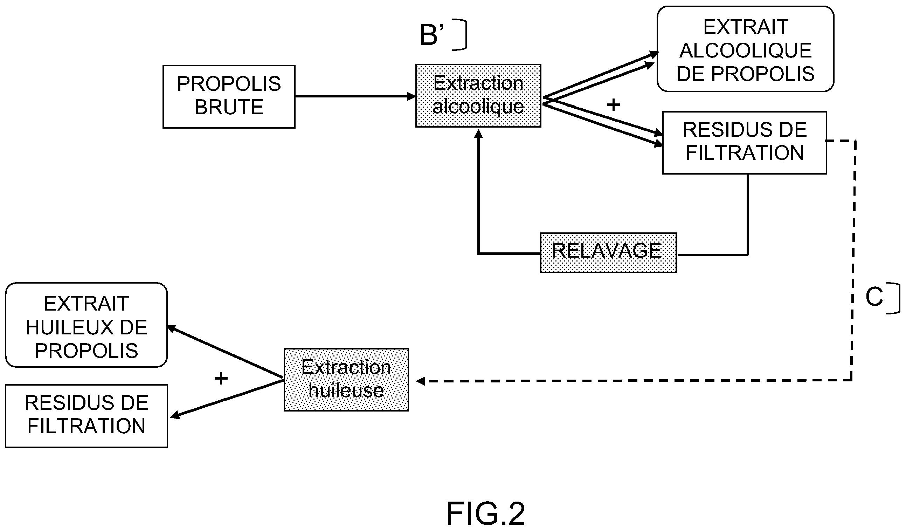

- la figure 2 représente les étapes mises en oeuvre selon un autre procédé ne faisant pas partie de l'invention,

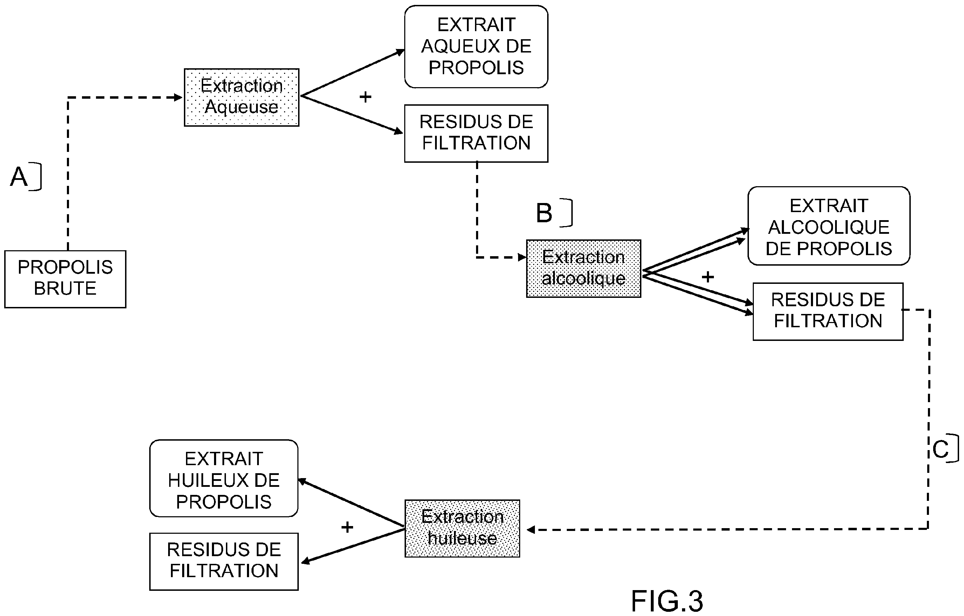

- la figure 3 représente les étapes mises en oeuvre selon le procédé de l'invention,
- la figure 4 représente les étapes détaillées d'une extraction aqueuse selon l'invention,
- la figure 5 représente les étapes d'une extraction alcoolique selon l'invention,
- la figure 6 représente les étapes d'une extraction huileuse selon l'invention.

Le procédé selon l'invention permet d'obtenir trois extraits de type différent (aqueux, alcoolique, huileux) pouvant être utilisés pour des applications différentes.

Ce procédé permet en outre d'obtenir à l'issue de chaque extraction des composantes actives différentes de la propolis. En effet, l'avantage que procure l'extraction avec de l'eau suivie d'une extraction avec de l'alcool est que l'extraction à l'eau ne prélève que des matières actives non solubles dans l'alcool et que la vapeur d'eau facilite la dissolution dans l'alcool des composantes actives solubles dans l'alcool. De la même façon en réalisant une extraction avec de l'alcool puis avec de l'huile, on n'obtient aucun déchet (pureté à 100%) et 100% des composantes actives.

A cette fin, le procédé comprend la mise en oeuvre de trois extractions successives opérées à partir d'un même lot de propolis brute (propolis brute pour la première extraction) et des résidus issus des extractions précédentes de propolis pour les deux autres extractions. Les trois extractions de propolis enchaînées ne sont pas réalisées sur des lots distincts.

En outre les extractions réalisées sont des extractions longues (dans le temps, ou encore lentes) par opposition à la pratique actuelle qui consiste à effectuer une extraction en quelques heures. Cette lenteur ainsi que l'absence de machines permet à des éléments subtils de mieux muter et d'éviter une altération de la structure physique de la propolis. Lors de la première étape, le procédé consiste à réaliser une extraction avec de l'eau pure, c'est-à-dire sans mélange avec un autre solvant. Cette extraction est effectuée sur un lot de propolis brute afin d'obtenir un premier extrait ayant des premières propriétés.

Pour des applications particulières, le procédé va à l'encontre des idées reçues car le solvant est de l'eau sans autre solvant ou additif. Le procédé permet ainsi d'obtenir un extrait aqueux qui possède des matières actives de la propolis lui conférant des propriétés antiseptiques, antibactériennes et isotoniques. Les applications sont par exemple la fabrication de déodorants, de produits d'hygiène nasale ou auriculaire.

L'extrait aqueux est réalisé par décoction de la propolis. On place la propolis dans de l'eau et on la porte à ébullition. On peut obtenir cet extrait en réalisant une infusion c'est-à-dire en laissant reposer la propolis dans l'eau portée à ébullition. On peut aussi réaliser une décoction puis laisser reposer la propolis dans l'eau portée à ébullition (infusion). Le procédé comporte une deuxième extraction de propolis avec de l'alcool pur. L'extraction avec l'eau est réalisée sur le lot de propolis brute et l'extraction avec l'alcool est réalisée sur les résidus de filtration obtenus à l'issue de l'extraction réalisée avec l'eau, de manière à obtenir le premier extrait de propolis ayant les premières propriétés puis un deuxième extrait ayant des deuxièmes propriétés, ce dernier étant débarrassé de la majorité des impuretés. Selon le procédé, on réalise successivement une extraction aqueuse puis une extraction alcoolique puis une extraction huileuse à partir d'un même lot de propolis brute.

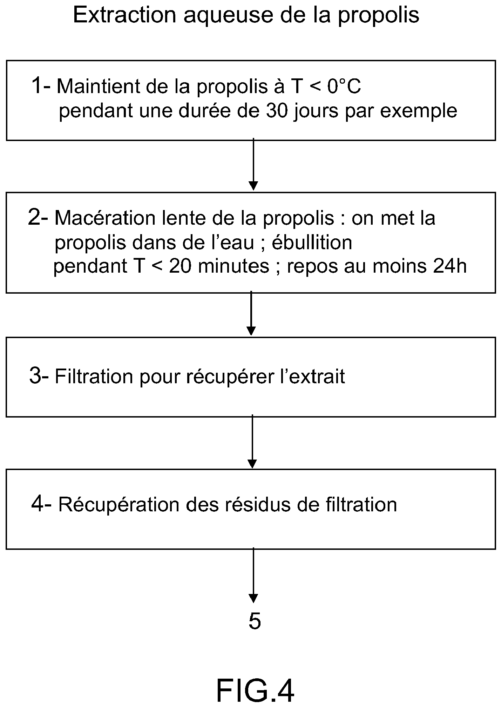

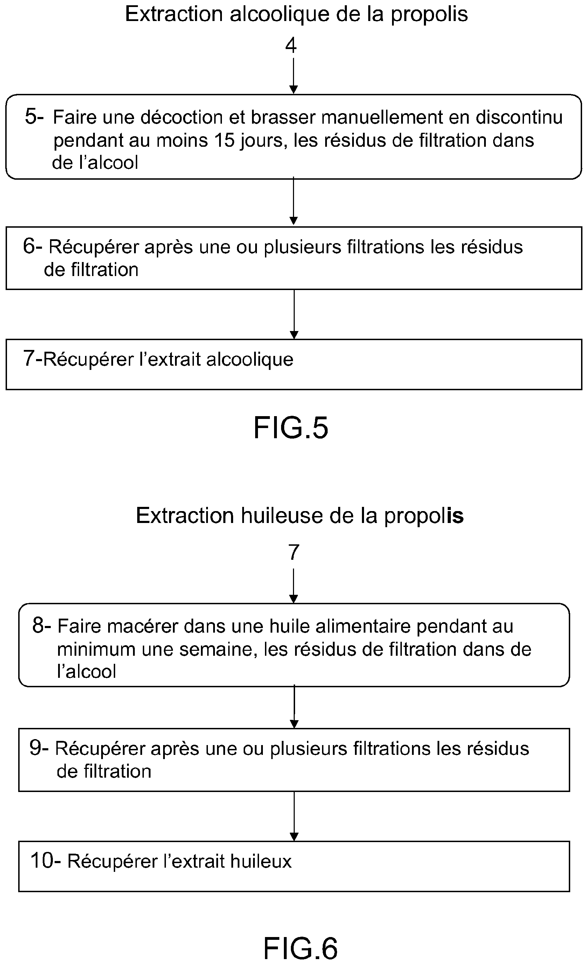

Dans un exemple particulier de réalisation on met en oeuvre les étapes suivantes :
A) Extraction aqueuse longue (figure 4):
   1- maintien de la propolis brute à une température inférieure à zéro degré pendant une durée relativement longue, 30 jours par exemple,
   2- Décoction de la propolis : on met la propolis dans de l'eau et on porte à ébullition pendant une durée ne dépassant pas 20 minutes,
   3- on laisse reposer au moins 24h pour récupérer l'extrait. Cette récupération est réalisée à l'issue d'une ou plusieurs filtrations,
   4- on récupère les résidus de filtration.
B) Extraction alcoolique longue (figure 5):
   Les résidus issus de l'étape 4 sont utilisés pour réaliser l'extraction alcoolique selon schéma de la figure 5.
   5- On fait macérer et on brasse manuellement en discontinu pendant au moins 15 jours, les résidus de filtration dans de l'alcool,
   6- On récupère après une ou plusieurs filtration(s) les résidus de filtration,
   7- On récupère l'extrait alcoolique obtenu.
C] Extraction huileuse (figure 6):
   8- On fait macérer dans de l'huile, les résidus de filtration récupérés à l'étape 6,
   9- On réalise une filtration ou une décantation,
   10- On récupère l'extrait huileux obtenu.

La présente invention permet ainsi d'obtenir un extrait aqueux, un extrait alcoolique et un extrait huileux ; - chaque extrait peut être utilisé de façon indépendante ou associé avec un autre, dans la fabrication de produits d'hygiène ou de santé ou cosmétiques, alimentaire ou complément alimentaire.

Quelques exemples d'applications sont donnés dans la suite. Ces applications ont fait l'objet de tests en laboratoires confirmant les propriétés énoncées et la tolérance sur le corps humain.

*Applications des extraits de propolis ou de complexe d'extraits de propolis obtenus selon le procédé décrit ci-dessus :*

### 1-Déodorant du corps (aisselles, poitrine, pieds et mains moites) et des zones intimes dénommé par le déposant : DEODORANT UNIVERSEL BIS.

Composition : 99,9 % d'extrait aqueux de propolis biologique, et 0, 1% d'élixir commercialisé par le déposant sous le nom d'Élixir de la Ruche Sueur du Ciel.

Différentes études ont été réalisées par l'Institut Dermatologique d'Aquitaine sur la formule ci-dessus, visant à évaluer :
a- l'acceptabilité cosmétique
b- la tolérance gynécologique sous le contrôle d'un médecin gynécologue
c- La tolérance cutanée sous le contrôle d'un médecin dermatologue.

Conclusions du rapport d'étude montrent que :
La formule présente :
- une bonne tolérance cutanée globale ;
- une acceptabilité cosmétique satisfaisante ;
- une bonne tolérance cutanéo-muqueuse.
   d- L'activité bactéricide sur les souches
   d1- Corynebacterium xerosis
   d2- Staphylococcus epidermidis
   d3- Propionibacterium acnes

### 2- Hygiène intime avec haute tolérance dénommé par le déposant : PAIN HYGIÈNE INTIME,

Composition : 94% pâte à savon biologique d'origine végétale (se trouvant dans le commerce), miel biologique (du commerce), 5% extrait alcoolique de propolis biologique obtenu selon le procédé, 1% élixir commercialisé par le déposant sous le nom d'Élixir de la Ruche Vol Nuptial.

Différentes études ont été réalisées par l'Institut Dermatologique d'Aquitaine sur la formule ci-dessus.
a- Évaluation de la tolérance cutanée après application unique sous pansement occlusif pendant 48 heures sous le contrôle d'un médecin dermatologue.

Conclusion du rapport d'étude: le PAIN HYGIÈNE INTIME appliqué dilué à 2%, peut être considéré comme **non irritant** après 48 heures consécutives d'application sous pansement occlusif chez 11 volontaires.
b- Évaluation de la tolérance gynécologique et des qualités cosmétiques après 21 jours d'application sous le contrôle d'un médecin gynécologue

Conclusions du rapport d'étude : le PAIN HYIÈNE INTIME présente une bonne tolérance cutanéo-muqueuse globale ainsi qu'une bonne acceptabilité cosmétique.

### 3- Soin après-rasage apaisant- micro-coupures - cicatrisant naturel

Produit dénommé par le déposant : **CRÈME DE FORCE**

Composition : émulsion (base aqueuse ou huileuse 76%) préparée avec un complexe d'extraits de propolis : 14% d'extrait huileux et 10 % d'extrait aqueux de propolis

Différentes études sont en cours de réalisation par l'Institut Dermatologique d'Aquitaine sur la formule ci-dessus afin de confirmer :
- Les propriétés citées ci-dessus (Soin après-rasage apaisant- micro-coupures - cicatrisant naturel)
- la tolérance cutanée après application unique sous pansement occlusif pendant 48 heures.
- la tolérance cutanée et des qualités cosmétiques après 21 jours d'application.
- La tolérance oculaire

### 4- Anti-oxydant - lisse les cernes et les ridules

Produit dénommé par le déposant : **YEUX DE REINE**

Composition : émulsion (base aqueuse ou huileuse 76%) préparée avec un complexe d'extraits de propolis : 14% d'extrait huileux et 10 % d'extrait aqueux de propolis.
- Les propriétés citées ci-dessus (Anti-oxydant - lisse les cernes et les ridules)
- la tolérance cutanée après application unique sous pansement occlusif pendant 48 heures.
- la tolérance cutanée et des qualités cosmétiques après 21 jours d'application.
- La tolérance oculaire

### 5- Anti-oxydant- hydratant et protecteur

Produit dénommé par le déposant : **LAIT DE RUCHE**

Composition : émulsion (base aqueuse ou huileuse 80%) préparée avec un complexe d'extraits de propolis : 8% d'extrait huileux et 12 % d'extrait aqueux de propolis

Différentes études sont en cours de réalisation par l'Institut Dermatologique d'Aquitaine sur les formules ci-dessus afin de confirmer :
- Les propriétés citées ci-dessus (Anti-oxydant - hydratation et protection)
- la tolérance cutanée après application unique sous pansement occlusif pendant 48 heures.
- la tolérance cutanée et des qualités cosmétiques après 21 jours d'application.

### 6-Cuir chevelu sain

- Démangeaisons du cuir chevelu
- Assainissement du cuir chevelu
- Anti-pelliculaire
- Anti-chutes des cheveux - alopécie
   → pour une chevelure saine et revigorée

Produit dénommé par le déposant : **CRÈME DOUCHE DE LA RUCHE**

Composition : 94% base lavante d'origine végétale, 1 % d'extrait alcoolique de propolis, 5 % de miel

Produit dénommé par le déposant : **SHAMPOING DOUCHE ASSAINISSANT ET DOUX.**

Composition : 94% base lavante d'origine végétale, 1 % d'extrait alcoolique de propolis, 5 % de miel

Produit dénommé par le déposant : **SHAMPOING SOIN RÉGÉNÉRANT.**

Composition : 97,5% base lavante d'origine végétale, 2,5 % d'extrait alcoolique de propolis

### 7- Hygiène nasale et auriculaire

### Application nasale

- Allergies et sensibilité saisonnière - action antivirale
- Nettoyage des résidus de pollution
- Hydratation de la muqueuse nasale

Produit dénommé par le déposant : **SPRAY NASAL DES PYRÉNÉES.**

Composition : préparation isotonique à base d'extraits de plantes 50% et d'extrait aqueux de propolis 50%

### Application auriculaire

- Bain d'oreille apaisant et nettoyant-antidémangeaisons.

Produit dénommé par le déposant : **EXTRAIT DE PROPOLIS SANS ALCOOL.**

Composition : préparation isotonique à base d'extrait aqueux de propolis (100%)

### 8- Hygiène dentaire et buccale

- Blanchissement naturel par effet antibactérien
- Parodontose
- Nettoyage des contours d'implants et de prothèses dentaires

Produit dénommé par le déposant : **DENTIFRICE SOURIRE**

Composition : préparation contenant 3,1 % d'extrait alcoolique de propolis et 96, 9 % d'un produit de base d'hygiène pour les dents du commerce.

Différentes études ont été réalisées par l'Institut Dermatologique d'Aquitaine sur la formule ci-dessus, dont l'évaluation de la tolérance et de l'acceptabilité après 21 jours d'application sous le contrôle d'un médecin stomatologue.

Conclusions du rapport d'étude : la formule présente une très bonne tolérance stomatologique ainsi qu'une bonne acceptabilité cosmétique.

## Revendications

1. Procédé de traitement de la propolis, **caractérisé en ce qu'**il comprend plusieurs étapes d'extractions successives opérées à partir d'un même lot de propolis, l'ensemble desdites étapes d'extraction étant réalisé sur une durée totale d'au moins 52 jours, lesdites étapes d'extraction étant réalisées strictement dans l'ordre suivant:
- A] une première étape d'extraction aqueuse, d'une durée de l'ordre de 30 jours à une température inférieure à 0°C, avec de l'eau pure, c'est-à-dire sans mélange avec un autre solvant, effectuée sur un lot de propolis brute afin d'obtenir un premier extrait aqueux ayant des premières propriétés antibactériennes, antiseptiques et isotoniques c'est-à-dire compatibles avec une application sur les muqueuses et applicable directement sur la peau,
- B] une deuxième étape d'extraction avec de l'alcool pur, ladite deuxième extraction alcoolique étant réalisée sur des résidus de filtration obtenus à l'issue de ladite première étape d'extraction aqueuse, par macération et brassage manuel discontinu desdits résidus de filtration aqueux pendant une durée d'au moins 15 jours, de manière à obtenir un deuxième extrait alcoolique ayant des deuxièmes propriétés,
- C] une troisième étape d'extraction avec de l'huile pure, ladite troisième extraction huileuse étant réalisée sur des résidus de filtration obtenus à l'issue de ladite deuxième étape d'extraction alcoolique, en les faisant macérer dans l'huile pendant au moins une semaine, de manière à obtenir un troisième extrait huileux ayant des troisièmes propriétés.

2. Procédé de traitement de la propolis selon la revendication 1, **caractérisé en ce que**, La première étape d'extraction aqueuse A) est une extraction aqueuse comprenant :
1- le maintien de la propolis à une température inférieure à zéro degré pendant une durée de l'ordre de 30 jours,
2- la décoction de la propolis : on met la propolis dans de l'eau et on porte à ébullition pendant une durée ne dépassant pas 20 minutes,
3- le repos au moins 24h pour récupérer l'extrait, cette récupération étant réalisée à l'issue d'une ou plusieurs filtration(s),
4- la récupération des résidus de filtration.
La deuxième étape d'extraction alcoolique B) est une extraction alcoolique comprenant :
5- la macération et le brassage manuel en discontinu pendant au moins 15 jours des résidus de filtration dans de l'alcool,
6- la récupération après une ou plusieurs filtration(s) des résidus de filtration,
7- la récupération de l'extrait alcoolique obtenu.
La troisième étape d'extraction huileuse C] est une extraction huileuse comprenant :
8- la macération dans de l'huile pendant au moins une semaine des résidus de filtration récupérés à l'étape 6,
9- une filtration,
10- la récupération de l'extrait huileux obtenu.

## Patentansprüche

1. Verfahren zur Behandlung von Propolis, **dadurch gekennzeichnet, dass** es mehrere aufeinanderfolgende Extraktionsschritte aus einer gleichen Propolischarge umfasst, wobei alle Extraktionsschritte über eine Gesamtdauer von zumindest 52 Tagen durchgeführt werden, wobei die Extraktionsschritte streng in der folgenden Reihenfolge durchgeführt werden:
- A] ein erster Schritt mit wässriger Extraktion mit einer Dauer von etwa 30 Tagen bei einer Temperatur unter 0 °C mit reinem Wasser, d. h. ohne Vermischung mit einem anderen Lösungsmittel, der mit einer Rohpropolischarge durchgeführt wird, um einen ersten wässrigen Extrakt zu erzielen, der erste antibakterielle, antiseptische und isotonische Eigenschaften aufweist, d. h. die mit einer Anwendung auf den Schleimhäuten kompatibel sind, und der direkt auf die Haut aufgetragen werden kann,
- B] ein zweiter Extraktionsschritt mit reinem Alkohol, wobei die zweite alkoholische Extraktion an Filterrückständen durchgeführt wird, die nach dem ersten wässrigen Extraktionsschritt erzielt wurden, und zwar durch Mazeration und diskontinuierliches manuelles Rühren der wässrigen Filterrückstände über einen Zeitraum von mindestens 15 Tagen, um einen zweiten alkoholischen Extrakt mit zweiten Eigenschaften zu erzielen,
- C] ein dritter Extraktionsschritt mit reinem Öl, wobei die dritte ölige Extraktion an Filterrückständen durchgeführt wird, die am Ende des zweiten alkoholischen Extraktionsschritts erzielt wurden, indem sie mindestens eine Woche lang in dem Öl mazerieren, um einen dritten öligen Extrakt mit dritten Eigenschaften zu erzielen.

2. Verfahren zur Behandlung von Propolis nach Anspruch 1, **dadurch gekennzeichnet, dass**,
der erste wässrige Extraktionsschritt A) eine wässrige Extraktion ist, die Folgendes umfasst:
1- die Erhaltung der Propolis bei einer Temperatur unter Null Grad für einen Zeitraum von etwa 30 Tagen,
2- die Propolisabkochung: Die Propolis in Wasser geben und nicht länger als 20 Minuten kochen lassen,
3- die Ruhezeit von mindestens 24 Stunden, um den Extrakt zu gewinnen, wobei diese Rückgewinnung nach einem oder mehreren Filtervorgängen durchgeführt wird,
4- die Gewinnung der Filterrückstände.
der zweite Extraktionsschritt B) eine alkoholische Extraktion ist, die Folgendes umfasst:
5- die Mazeration und das diskontinuierliche manuelle Rühren der Filterrückstände in Alkohol an mindestens 15 Tagen,
6- die Gewinnung nach einem oder mehreren Filtervorgängen der Filterrückstände,
7- die Rückgewinnung des erzielten alkoholischen Extrakts.
der dritte Extraktionsschritt C] eine ölige Extraktion ist, die Folgendes umfasst:
8- die Mazeration der im Schritt 6 gewonnenen Filterrückstände über mindestens eine Woche,
9- einen Filtervorgang,
10- die Rückgewinnung des erzielten öligen Extrakts.

## Claims

1. A method to treat propolis, **characterized in that** it comprises several steps of successive extractions conducted on the same batch of propolis, all of said extraction steps being performed over a total period of at least 52 days, said extraction steps being performed strictly in the following order:
- A] a first step of aqueous extraction, lasting about 30 days at a temperature below 0°C, with pure water, i.e., without mixing with another solvent, carried out on a batch of raw propolis in order to obtain a first aqueous extract having first antibacterial, antiseptic and isotonic properties, i.e., compatible with application to the mucous membranes and applicable directly to the skin,
- B] a second step of extraction with pure alcohol, said second alcohol extraction being performed on the filtration residues obtained after said first step of aqueous extraction, by maceration and discontinuous manual mixing of said aqueous filtration residues for a period of at least 15 days, so as to obtain a second alcohol extract having second properties,
- C] a third step of extraction with pure oil, said third oil extraction being performed on the filtration residues obtained after said second step of alcohol extraction, by leaving them to macerate in the oil for at least one week, so as to obtain a third oil extract having third properties.

2. The method to treat propolis of claim 1, **characterized in that**,
The first step of aqueous extraction A) is an aqueous extraction comprising:
1- holding the propolis at a temperature below zero degrees for a period of about 30 days,
2- decocting the propolis: the propolis is placed in water and brought to the boil for a time not exceeding 20 minutes,
3- leaving the propolis to stand at least 24h to collect the extract, this collection being performed after one or more filtration(s),
4- collecting the filtration residues.
The second step of alcohol extraction B) is an alcohol extraction comprising:
5- macerating the filtration residues in alcohol and discontinuously manually mixing for at least 15 days,
6- after one or more filtration(s), collecting the filtration residues,
7- collecting the alcohol extract obtained.
The third step of oil extraction C] is an oil extraction comprising:
8- macerating the filtration residues collected at step 6 in oil for at least one week,
9- one filtration,
10- collecting the oil extract obtained.
